# EUROPEAN PATENT SPECIFICATION

(11) **EP 0 473 697 B1**
(45) Date of publication and mention of the grant of the patent: **14.08.1996**
(21) Application number: 90908859.3
(22) Date of filing: 22.05.1990
(51) Int. Cl.: A61M 25/00

(54) **CATHETER WITH LOW-FRICTION DISTAL SEGMENT**
KATHETER MIT EINEM REIBUNGSARMEN DISTALEN SEGMENT
CATHETER AVEC SEGMENT DISTAL DE FAIBLE FRICTION

(30) Priority: 22.05.1989 US 355500
(43) Date of publication of application: 11.03.1992
(73) Proprietor: TARGET THERAPEUTICS, INC., Fremont, CA 94537-5120 (US)
(72) Inventor: ENGELSON, Erik, T., Mountain View, CA 94041 (US); SEPETKA, Ivan, Redwood City, CA 94063 (US)
(74) Representative: Senior, Alan Murray
(86) International application number: PCT/US90/02791
(87) International publication number: WO 90/14123

(56) References cited:
- WO-A-87/07493
- GB-A- 3 531
- US-A- 3 416 531
- US-A- 4 044 765
- US-A- 4 636 346
- US-A- 4 705 511
- US-A- 4 737 153
- US-A- 4 863 442

## Description

The present invention relates to an improved catheter and catheter device for accessing a tissue target site along a tortuous or highly curved path through small vessels. More particularly, the invention relates to a catheter comprised of a tube and a guidewire wherein the internal surface of at least a portion of the tube is designed so as to facilitate the relative movement of the guidewire with respect to bent or curved portions of the tube and thus prevent jamming, sticking or locking of the guidewire against the internal tube surface.

Catheters are being used increasingly as a means for delivering diagnostic or therapeutic agents to internal target sites that can be accessed through the circulatory system. For example, in angiography, catheters are designed to deliver a radio-opaque agent to a target site within a blood vessel, to allow radiographic viewing of the vessel and blood flow radiographic viewing of the vessel and blood flow characteristics near the release site. For the treatment of localized disease, such as solid tumors, catheters allow a therapeutic agent to be delivered to the target site at a relatively high concentration with minimum overall side effects.

Often the target site which one wishes to access by catheter is buried within a soft tissue, such as brain or liver, and can only be reached by a tortuous route (i.e., a route including repeated sharp curves) through small vessels or ducts --typically less than about 3 mm lumen diameter-- in the tissue. The difficulty in accessing such regions is that the catheter must be quite flexible in order to follow the tortuous path into the tissue, and at the same time, stiff enough to allow the distal end of the catheter to be manipulated from an external access site, which may be as much as a meter or more from the tissue site.

Heretofore, two general methods for accessing such tortuous-path regions have been devised. The first method employs a highly flexible catheter having an inflatable, but pre-punctured balloon at its distal end. In use, the balloon is partially inflated and carried by blood flow into the target site. The balloon is continually inflated during placement to replenish fluid leaking from the balloon. A major limitation of this method is that the catheter will travel in the path of highest blood flow rate, so many target sites with low blood flow rates cannot be accessed.

In the second method, a torqueable guidewire and catheter are directed as a unit from a body access site to a tissue region containing a target site. The guidewire is bent at its distal end and may be guided, by rotating and advancing the wire, along a tortuous, small-vessel pathway, to the target site. Typically the guidewire and catheter are advanced along the tortuous pathway by alternately advancing the wire along a region of the pathway, then advancing the catheter axially over the advanced wire portion. An important advantage of this method is the ability to control the location of the catheter along a tortuous path.

It is frequently desirable, for example, in treating deep brain vessel abnormalities, to direct a small-diameter catheter along a tortuous, small-diameter pathway to the brain vessel site. The procedure may be advisable, for example, in treating an arteriovenous malformation, in order to introduce an embolic agent into the small capillaries connecting the arterial and venous vessels at a deep brain site. At a certain point along the pathway, when sharp bends are first encountered, the catheter is advanced by alternately guiding the flexible-tip portion of the guidewire along the path, then threading the catheter over a portion of the advanced wire region.

One problem which may be encountered, as the guidewire and catheter are advanced, is that the guidewire can become stuck against the internal tubular surface of the catheter. Typically, this problem arises when a sharp bend, such as a hairpin loop, is encountered and/or where two or more sharp bends occur in succession. When the catheter and wire become locked together (i.e., the end of the guidewire is jammed against the internal surface of the catheter tube so as to prevent the relative movement of the guidewire and internal tubular surface) in the region of wire bending, it may be impossible to either advance or withdraw the wire. In this event, the wire and catheter must be pulled back as a unit along the pathway until both are straight enough to allow the wire to be moved axially within the catheter, and often, the physician may have to give up attempting to reach the site.

The problem of advancing a catheter over a guidewire in a region of sharp wire bend(s) has been addressed by the catheter construction disclosed in WO-A-87/07493, on which the preamble of claim 1 is based. This construction includes a relatively long, relatively rigid proximal segment, and a shorter, more flexible distal segment having a length of at least about 5cm. The proximal segment provides sufficient torqueability and axial stiffness for guiding the catheter and internal guidewire from a body access site to the target tissue of interest. Once the tortuous tissue pathway is reached, the more flexible end segment allows the end region of the catheter to be advanced over sharp and/or frequent wire bends.

US-A-4 636 346 discloses a guiding catheter for providing a guiding lumen through which a treatment catheter, such as an intravascular catheter or the like, may be advanced. The guiding catheter is for use in conjunction with a guidewire and has a first section, towards its proximal end, of substantially less flexibility relative to a second positioned towards the distal end.

According to the present invention there is provided a catheter for use in combination with a guidewire for accessing a target site in an internal body tissue, from an external body site to the internal body tissue, and along a tortuous, small-vessel pathway within the tissue, said catheter comprising:
an elongate tubular member having proximal and distal ends, and an inner lumen extending between these ends, the lumen having a diameter which is no greater than about 0.1cm (40 mils), said member including a relatively rigid proximal segment and a relatively flexible distal segment at least about 5cm long which is adapted for tracking the guidewire along such tortuous path, said distal segment including a flexible, relatively deformable, polymer distal-segment tube;
characterised in that the distal segment further includes a relatively non-deformable internal surface means carried on the inner surface of the distal-segment tube, for providing substantially uninterrupted reduced friction contact with the guidewire, as the distal segment of the catheter is advanced over a looped or bent region of the guidewire.

In one preferred embodiment the surface means includes a sleeve which may, for example, be a braided-filament sleeve or a wire coil. The sleeve may comprise an anti-friction material.

In a further preferred embodiment the surface means includes an array of substantially non-deformable smooth-surfaced particles carried on the inner surface of the distal-segment tube. These particles may, for example, be carbon particles attached to the inner surface of the distal-segment tube by a binder.

In a yet further preferred embodiment the surface means includes a thin-walled extension of the proximal segment forming an inner coaxial lining of the distal segment.

In another preferred embodiment the surface means includes a chemically hardened polymer surface coat formed by cross-linking the inner wall surface of the distal segment.

The hereinafter described and illustrated embodiments of catheter include a tubular member with a highly flexible section which includes an internal wall member modified in a variety of different manners in order to reduce the potential for jamming, sticking or locking the distal end, or some other portion of the guidewire, against the inner surface of the distal-segment tube by rendering the distal segment capable of deflecting the guidewire from applying significant forces in a direction normal to the internal surface of the tubular wall. The embodiments of catheter can thus be used to enter highly curved areas with substantially reduced problems with respect to the jamming of the guidewire against the internal surface of the distal-segment tube.

Embodiments of apparatus in accordance with the present invention will now be described, by way of example only, with reference to the accompanying drawings, in which:
Figure 1 shows a catheter device, including a catheter constructed according to the present invention;
Figure 2 is an enlarged, sectional view of the catheter, taken along the region 2-2 in Figure 1, in an embodiment in which the internal tubular surface is a braided sleeve;
Figure 2A is the same view as Figure 2 showing the wire coil in place of the braided sleeve;
Figure 3 is a cutaway view of a portion of the distal-end segment of a catheter similar to the one shown in Figure 2, but where the braided sleeve has a reduced density and increased radial pitch on progressing toward the catheter's distal end;
Figures 4A and 4B are enlarged sectional views of a catheter constructed according to a second general embodiment, showing a distal-end segment of a catheter having a carbon-particle coating (4B) formed by drying a carbon slurry on the wall of the distal segment (4A);
Figure 5 is an enlarged, sectional view of a catheter constructed according to a third general embodiment, where the distal-end segment includes a thin-walled, relatively stiff inner lining or coat and a thicker-walled, relatively more flexible outer tube;
Figure 6 is a view of a catheter embodiment like that shown in Figure 5, but where the thickness of the inner tube in the distal-end segment is tapered on progressing toward the distal end of the catheter;
Figure 7 is an enlarged sectional view of a catheter device constructed according to a fourth general embodiment, where the distal-end segment has a chemically hardened inner coating;
Figure 8 illustrates an enlarged, fragmentary sectional view of one preferred type of guidewire for use in the catheter of the invention;
Figure 9 shows a test configuration for measuring the resistance of a catheter being advanced over a helically- wound wire;
Figure 10 shows plots of the force required to advance a standard polymer-tube catheter (dashed lines) and a catheter constructed according to the invention (dash-dot lines) over a smooth-surface wire, as a function of the position of the catheter on the Figure 9 helical wire turns;
Figure 11 illustrates a typical small-vessel pathway in which a serpentine configuration like that illustrated in Figure 11 is encountered; and
Figure 12 is an enlarged cross-sectional view of the catheter of the invention being advanced over a serpentine portion of a guidewire.

Before the present catheter, catheter device and process for using such is described, it is to be understood that this invention is not limited to the particular catheter devices, components, constructions and materials specifically recited as such may, of course, vary. It is to be understood that the terminology used herein is for purposes of describing particular embodiments only, and is not intended to be limiting since the scope of the present invention will be limited only by the appended claims.

It must be noted that as used in the specification and claims, the singular forms "a", "an" and "the" include plural referents unless the context clearly dictates otherwise. Thus, for example, reference to "a coiled construction" includes a plurality of such constructions, reference to "an anti-friction material" includes a plurality of such materials and reference to "the bending" includes reference to a plurality of bends made by the catheter and/or guidewire and so forth.

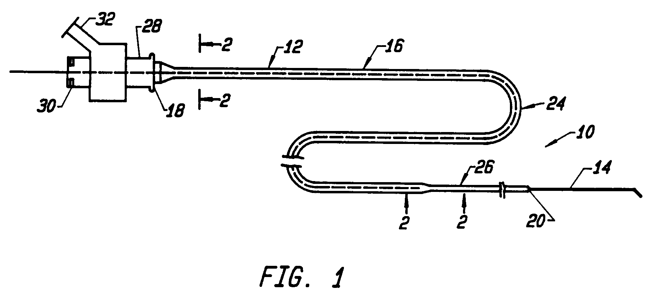

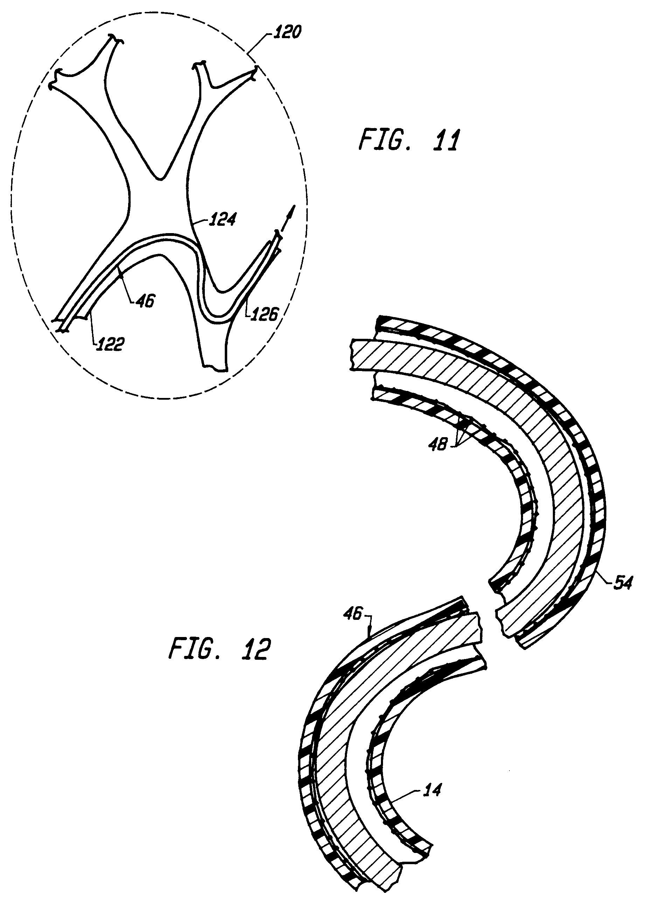

Figure 1 shows a catheter device 10 constructed according to the present invention. The device includes a catheter 12 in the form of an elongated tubular member which will be described below, and a guidewire, here indicated at 14. The catheter device is designed for accessing a target site which can be reached only along a small tunnel-like tortuous path within a target tissue, as will be described with reference to Figures 11 and 12 below.

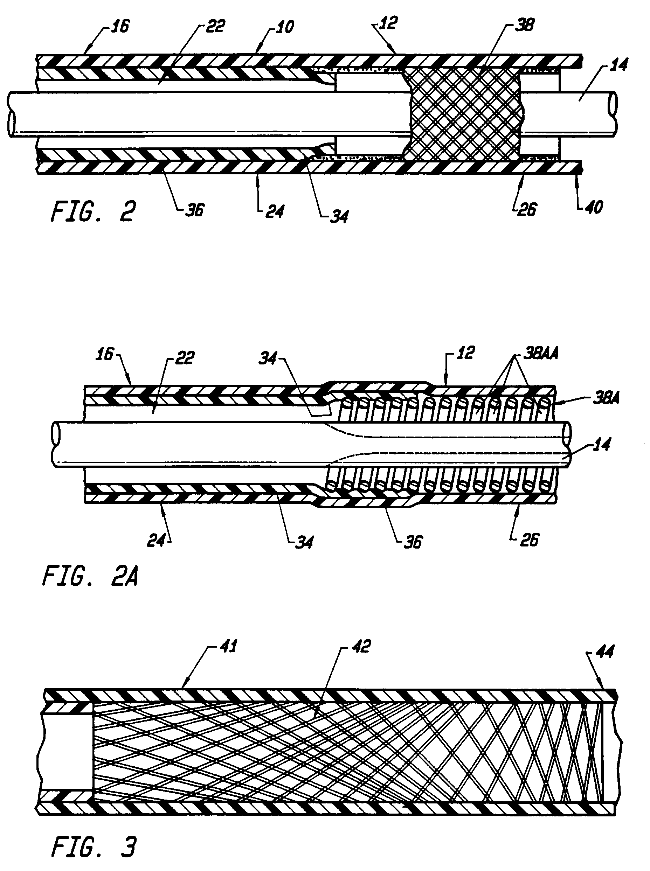

With continued reference to Figure 1, the catheter 12 includes an elongate outer tubular surface 16 having proximal end 18 connected at a fitting and a distal end. The tubular member 12 can be between about 50-300 cm in length, and is typically and more preferably between about 100-200 cm in length. The hollow cylindrical area inside the tube 12 or inner lumen 22 (Figure 2) extending between the two ends has a preferred diameter of less than about 0,1 cm (40 mil), and preferably between about 0,03-0,08 cm (12-30 mil). One mil is one thousandth of an inch, i.e., 0.001 inch. In one embodiment, the diameter of the inner lumen is between about 0,005-0,02 cm (2-7 mils) greater than that of the diameter of the guidewire 14 carried within the catheter 12. The lumen 22 may have a substantially uniform cross-sectional area along its length, or may vary along the catheter length, for example, the distal end may taper toward a small diameter in a direction away from the proximal end.

As will be described in greater detail below, the catheter or tubular member 12 includes a relatively stiff proximal segment or segment means 24 (a first section) terminating proximally at end 18, and a relatively more flexible distal segment or segment means 26 (a second section) terminating distally at end 20. Thus the first segment or proximal segment 24 has greater structural integrity, a greater resistance to bending, and is more stiff than the second or distal segment 26 which has less structural integrity, greater flexibility, and less resistance to bending than the first section. Although either segment can be comprised of a variety of materials it is important that the materials be modified and/or structured so as to obtain the desired differential with respect to the flexibility of the two sections. The greater stiffness and less flexibility of the first section 24 relative to the softer or more flexible material of the second segment 26 can be measured quantitatively by the bending forces necessary to bend either segment through an equivalent angle. The distal segment is at least about 5 cm long, with the proximal segment providing the remainder of the length of the catheter tubular member. Typically, the proximal segment makes up between about 70%-90% of the total length of the tubular member, and the relatively flexible distal segment makes up the remaining 10%-30% of the length.

Throughout this disclosure, the first or proximal section of the catheter will be referred to as stiffer or less flexible than the second or distal section of the catheter which will be referred to as more flexible and bendable than the first or proximal section. The degree of stiffness, flexibility and/or bendability can be measured quantitatively using tests known to those skilled in the art such as the American Society for the Standard of Testing of Materials (hereinafter referred to as ASTM). In connection with the present invention, the materials used in the catheter were tested using ASTM D747. It should be pointed out that ASTM D747 is generally used in connection with the testing of rectangular pieces of material. Since the present invention is in the form of a tubular catheter, the D747 test was modified for use in connection with the testing of tubular pieces of material. For purposes of this disclosure the ASTM test designated a D747 is incorporated herein by reference for purposes of disclosing methods of testing material with respect to their flexibility.

The above referred-to ASTM D747 modified test was carried out in connection with sections of tubular material to be used for the first or proximal section of the catheter device. The proximal or first section tested under modified D747 testing procedures should give a result of 1050 bar (15,000 psi) or more. Results as high as 4200 bar (60,000 psi) or more are possible. However, it is more likely that the results will yield a reading of about 2800 bar (40,000 psi) or more and are most preferably in the range of about 1750-2450 bar (25- to 35,000 psi) with one particular embodiment providing a result of 2030 bar (29,000 psi).

Tubular segments of material to be used in connection with the second section or distal section of the catheter were also tested using the ASTM modified D747 testing procedure. These more flexible or bendable segments gave a reading of 700 bar (10,000 psi) or less and are generally in the range of about 490-210 bar (7,000 to 3,000) psi. Although some particularly flexible tubings may have readings below 210 bar (3,000), e.g., about 700 bar (1,000 psi), a particularly preferred material has a reading of about 385 bar (5,500 psi).

Based on the above information, it can be seen that the most flexible D747 reading for the stiffer material 1050 bar (about 15,000 psi) is substantially greater than the least flexible material to be used in connection with the more flexible distal end (about 700 bar (10,000 psi) or less). In general, the modified D747 test reading for the stiffer or proximal section is at least 50 percent greater than the reading for the more flexible or distal segment, and is more preferably more than 100 percent greater. When given in terms of ranges, it can be pointed out that the stiffer or proximal segment is in the range of 2 to 30 times the D747 reading of the more flexible section and more preferably in the range of about 3 to 8 times greater than the D747 reading of the more flexible section.

The inner surface wall of the distal segment 26 of the catheter is constructed such that or comprised of a material such as a low-friction coat which allows the guidewire to be moved axially within the catheter through regions of sharp bends or turns. Four general embodiments of the internal surface of the distal segment are described below in Sections A-D.

The catheter device further includes a proximal end fitting 28 through which the guidewire is received, and through which fluid material can be introduced into the catheter lumen. One standard fitting which is suitable has a guidewire O-ring seal 30 which can be compressed to provide a suitable seal about the guidewire, while still allowing the wire to be rotated (torqued) and advanced or retracted axially within the catheter, during a catheter placement operation. Fluid material can be introduced into the catheter lumen, for example, from a syringe, through port 32.

### A. Catheter With Flexible-Sleeve Distal Segment

Figure 2 shows an enlarged cross sectional view of a region of catheter 10 in the region of transition between the two segments, indicated at 2-2 in Figure 1. As seen, proximal segment 24 is composed of inner 34 and outer 36 coaxial tubes which are tight-fitting and/or essentially integral with respect to each other. The stiffness in the proximal segment 24 is provided predominantly by an additional coaxial tube 34. The inner, stiffer tube 34 is preferably polypropylene or high-density polyethylene tubing having a wall thickness of between about 0,005-0,01 cm (2-4 mils). The outer, more flexible tube is preferably low density polyethylene or silicone tubing, also having a preferred wall thickness of between about 0,005-0,01 cm (2-4 mils). As defined herein, high- and low-density polyethylene have the usual trade meaning which is applied to the density grade of polyethylenes which are commonly used in extrusion. With respect to the present invention it is not critical that the materials be low and/or high density polyethylenes or silicon material. Any materials having differing properties of the type described above can be used to make up the two different tubular members. What is important is that the outer tube be comprised of a material of less stiffness and structural integrity and greater flexibility relative to the inner tube which is comprised of a material of greater structural integrity and stiffness and less ability to bend relative to the outer tube. By comprising the tubes of the different types of materials it is possible to include the first segment which is stiffer and less bendable and the second segment which is flexible and more easily bendable relative to the first segment.

It will be recognized that other tubing materials whose wall thickness can be adjusted to give comparable tubing flexibilities will be suitable, with the constraint that the total wall thickness of the proximal segment should be less than about 0,03 cm (10 mils), and that the number of tubing layers of constant or varying flexibility forming the segments, or portions thereof, can be varied to achieve desired flexibility properties in the tube. As an example, the proximal and distal segments may each be formed as a single layer tube, and joined together at the interface by suitable chemical adhesion and/or by overlapping the two tubes in a short interface region.

In the specific embodiment shown in Figure 2, the low-friction surface coat in the catheter is provided by a flexible braided sleeve 38 formed by braid-weaving relatively hard filament material, such as metal, nylon, or filaments of Teflon-like materials. The sleeve may be made by conventional braid-weaving methods, such as described in US-A-4,870,887 (disclosing such brands and their method of production), where the density and radial pitch of the braid may be varied according to the weave conditions.

In one preferred embodiment, the filaments used in making the sleeve are very fine platinum filaments, and the sleeve is woven under conditions which produce a loose weave having a radial pitch, defined by the angle of the weave with respect to the radial direction in the sleeve, of between about 20 - 60°.

The catheter can be constructed, according to one method, by anchoring the proximal end of sleeve 38 between the tubes 34 and 36 forming the proximal segment, as shown in Figure 2. The outer tube is preferably a heat-shrink material which is placed over the over the sleeve and inner tube and heat shrunk to form a snug fit over both the inner tube and sleeve 38. The sleeve is now anchored at the proximal end and tightly encased along its length by the distal-segment tube.

In the embodiment of the invention shown in Figure 2, the distal segment includes a distal extension 40 which extends beyond the end of sleeve 38. That is, the second or distal segment 26 includes (a) a proximal region having an inner tubular wall surface in which the frictional coefficient between that surface and the guidewire is significantly reduced, and (b) a region where this wall surface provides higher friction, but is an overall lower-mass tip region whose inertial mass is more closely matched with that of the tapered distal region of the guidewire. The latter feature reduces the tendency of the catheter to force the guidewire out of a bent or curved condition as the catheter is advanced over the distal end region of the guidewire. Explaining further, a relatively low mass at the distal tip region of the catheter may be necessary for tracking the catheter along a tapered region of the guidewire through a sharp bend or turn, even though the reduced catheter mass smaller is gained at the expense of increased friction in this tip region.

Alternatively, the inertial mass in the distal segment of the catheter can be reduced by decreasing the mass of the sleeve, either by employing a lighter or thinner sleeve filament or by reducing the density of filament(s) in the sleeve. It will be appreciated from Sections B-D below that several embodiments of the present invention provide a low-mass surface coating which combines reduced frictional coefficient with very little increase in the mass of the distal segment. The present invention contemplates a distal segment having combined types of low-friction coat, for example, a sleeve coat along a proximal region of the distal segment and one of the lower-mass low-friction coats described in sections B-D along the most distal region of the distal segment.

Figure 3 shows an alternate embodiment of a sleeve catheter 41, in which the braided sleeve, here indicated at 42, has a continually reduced pitch and weave density in a direction progressing toward the distal tip 44 of the catheter (toward the right in Figure 3). The sleeve 42 may be formed by conventional braiding methods, where the angle of filaments taken into the braid weave, and the rate of filament taken into the weave, is varied during weaving to obtain the desired change in braid pitch and density. Moving alone the sleeve 42 to the right it can be understood that the sleeve provides greater flexibility, as well as reduced mass, on progressing distally.

In the above-described embodiments, the internal tubular surface of the distal portion of the catheter is provided with structural features in the form of different types of braided sleeves. These structural features aid in preventing the jamming, sticking or locking of the internal surface of the catheter against an external surface of a guidewire. Other structural embodiments are possible. More specifically, the internal surface of the distal portion of the catheter can be constructed to include other structural features different from the braided sleeve which achieves similar results. What is important is that the structural features provide some ability to deflect normal forces which will be applied by the guidewire against the internal tubular surface so as to prevent jamming, sticking or locking of the guidewire against the surface.

Another embodiment of the invention (shown in Figure 2A) in which the braided sleeve described above is replaced with a wound-filament coil. This embodiment has been described in US-A-4,955,862. The coil 38A is preferably formed of a radio-opaque material, such as platinum, and has a pitch of which is preferably between 1.2-2 times the thickness of the filament. The coil 38A may be positioned like the braided sleeve 38 and may be encased in the distal polymer tubing, for example, by heat-shrinking the tube about the coil, as described above.

The sleeve 38 or 38A may be formed of a radio-opaque filament material, such as platinum, or may be plated or coated with a radio-opaque material, such as gold, for a fluoroscopic viewing.

Both the above-described braided sleeve 42 and the wound-filament coil 38A described in US-A-4,955,862 provide a structural element which aids in deflecting the distal end or some portion of the distal end of the guidewire from being jammed, stuck or locked against the internal tubular surface. Both the coils and the braided sleeves can be constructed in a variety of different manners in order to obtain the desired results of preventing or alleviating the presentation of substantial forces from the distal portion of the guidewire in a direction normal to the surface of the internal tube. Further, both the coils and the braiding can be constructed of a variety of different materials. It is preferable if the coils and/or braiding are comprised of materials so as to reduce as much as possible the frictional resistance between the internal surface of the tubing and the external surface of the guidewire. Reduced friction aids in preventing the jamming or locking of the distal end or any part of the second section of the guidewire against the internal surface of the tube, that is, against the outer surface of the braiding or coiling component.

### B. Discrete-Particle Coating

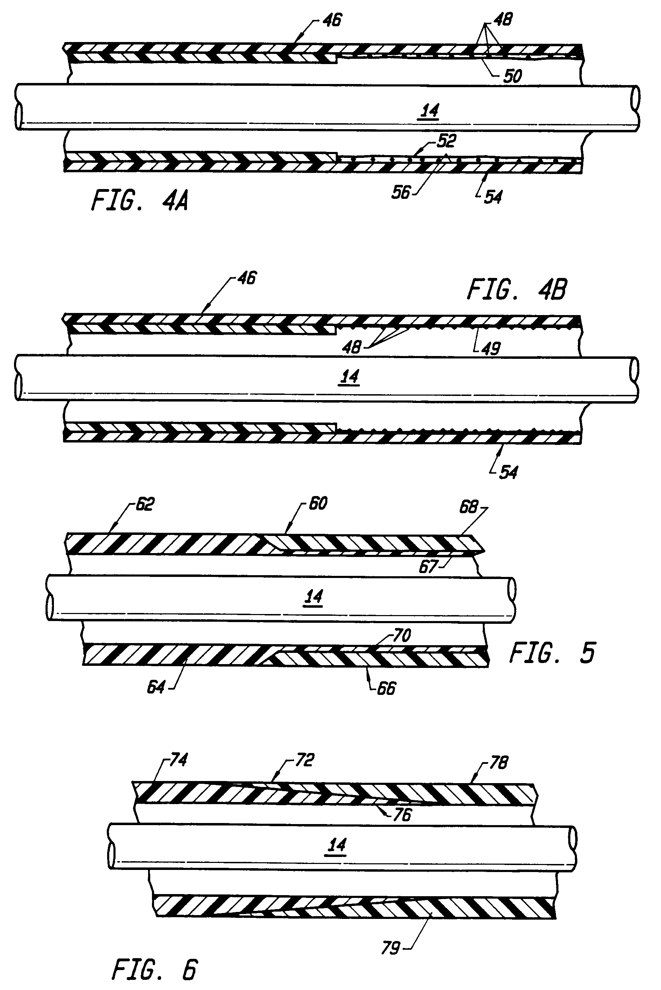

In a second general embodiment (see Figure 4A and 4B), the low-friction surface coat on the distal segment of the catheter is provided by means of a plurality of low-friction particles embedded in or otherwise attached to the inner wall of the distal segment tube 26. An embodiment of the invention in which the coating includes spherical metal particles embedded in the inner wall of the distal-segment tube has been described in US-A-4 955 862. Briefly, metal spheres having preferred diameters between about 0,003-0,008 cm (1-3 mils) are embedded in the distal segment 26, e.g., as the tube is being extruded.

The particles are provided in sufficient number such that the surface density of the particles insures substantially uninterrupted contact between the spheres and a guidewire, when the distal segment is advanced over a looped or sharply bent portion of the guidewire. Preferably the particle density and size is such as to produce sphere-to-sphere contact when the catheter is moved toward a sharply bent configuration. The particle density acts to resist catheter bending beyond the point where the particles are brought into contact with one another, thus serving to prevent kinking in the catheter.

An alternative embodiment of a catheter 46 having a discrete-particle coating is illustrated in Figures 4A and 4B. Here the particles 48 are carbon, preferably graphitic, particles which are deposited on the inner wall of the distal segment in a suitable binder. In one preferred method graphite particles 48, such as particles 48 in Figure 4A, in the 0,0013-0,005 cm (1/2 - 2 mil) size range are suspended in a liquid resin mixture 50, such as a mixture of unpolymerized or partially polymerized polyurethane or phenolic resin in the presence of a suitable catalyst, to form a particle slurry 52. The slurry is introduced into a distal catheter segment 54 to coat the inner wall 56, as illustrated in Figure 4A.

The slurry binder is polymerized under solvent-removal conditions, typically by placing the catheter under vacuum and rotating the segment during solvent removal to maintain an even coat during drying. Plasma treatment technologies can be used. The final binder coat is indicated at 49 in Figure 4B.
Methods for preparing and hardening resin mixtures which can be polymerized at relatively low temperatures are known. The distal-segment tube may be mechanically or chemically abraded prior to coating, to produce improved bonding of the binder layer to the wall of the tube. Alternatively, chemical bonding agents can be incorporated into the binder to bond the polymer in the binder to the segment wall. Such bonding agents are well-known, e.g., as described in US-A-3,698,931 incorporated herein by reference to disclose such bonding agents.

Alternatively, the carbon particles may be embedded in the inner wall of the distal-segment tube during extrusion, as described above with respect to metal particles.

It will be appreciated that other low-friction particles, such as anti-stick Teflon-like material or nylon beads may be employed in forming the discrete-particles surface coating. An advantage of carbon or polymeric beads over metal beads is their lighter weight, which allows better trackability over the tapered region of the guidewire, for the reasons discussed above. The carbon or polymer-bead particles may be plated with a radio-opaque material, such as gold, for fluoroscopic viewing.

Including the particles on the inner surface of the tubular wall provides structural features which aid in deflecting the distal or second section of the guidewire so as to prevent the guidewire from applying substantial forces normal to the surface of the tubular wall and thus prevent sticking, jamming or locking the distal portion of the guidewire against the inner wall surface. In addition to providing the structural features which deflect the guidewire the particles can be comprised of the above-referred to anti-friction materials which facilitate the movement of the distal end of the guidewire along the inner tubular surface. Thus, like the braided member embodiment described above, the particle coating embodiment described in this section can include both structural features for deflecting the guidewire and composition (i.e., anti-friction material) features for reducing friction, both of which provide the desired result of the invention which is to avoid to the greatest extent possible the jamming, locking or sticking of the guidewire against the inner tubular surface when the tube and guidewire are bent repeatedly while being moved through a tortuous course.

### C. Coextruded Polymer Surface Coating

In a third general embodiment, illustrated in Figures 5 and 6, the low-friction surface coating in the distal segment of the catheter is provided by a relatively hard-surfaced polymer tube. One catheter having this general construction is shown at 60 in Figure 5. As in the earlier-described embodiments, the catheter includes a first or proximal segment 62 formed of a relatively stiff, non-deformable polymer tube 64, such as high-density polyethylene, polyurethane, polypropylene, or Teflon-like material as described above, and a distal segment 66 formed of an outer, relatively flexible, deformable polymer tube 68, and an inner distal extension of tube 64.

As seen, tube 64 is sharply reduced in wall thickness at the transition zone between the two segments, yielding a thin-walled tube section 70 which forms the inner surface 67 of segment 66. Typically, the wall thickness of the inner tube is between about 0,0013-0,0026 cm (0.5-1 mil), and about 10-20 % of the wall thickness of the outer tube 68. The surface 67 can include structural features or be comprised of materials which together or alone prevent jamming of the guidewire 14.

The catheter can be formed by coextrusion, according to known tube co-extrusion methods, such as detailed in US-A-4,680,156 and 4,499,041, each of which are incorporated herein by reference to disclose such methods. The extrusion conditions are adjusted to extrude (a) a single-layer section of relatively rigid polymer tube within a more flexible outer tube, (b) in a transition zone in which the wall-thickness of the inner tube is reduced, and (c) a distal segment in which the inner tube has a fixed, reduced wall thickness.

Alternatively, the catheter may be constructed by first forming the single proximal tube with the reduced-thickness distal extension, then covering the distal extension with a relatively flexible distal tube. The proximal tube and its thin-walled extension can be formed, according to one method, by extrusion processing known to those skilled in the art, or by thinning an end section of the extruded tube by heating and stretching the end portion. In still another approach, a constant-thickness proximal tube is machined in its distal region to form the thin-walled portion.

The outer, more flexible tube in the distal section can be formed over reduced-thickness inner tube section by heat-shrinking the outer tube over the inner one, by binding the two tubes, or by coating the inner tube with a flexible-polymer outer coating, such as by conventional dipping or spraying methods. Figure 6 illustrates a catheter 72 in which the proximal tube, indicated at 74, has a gradual taper in a proximal region 76 of a distal segment 78. The outer, more flexible tube, shown at 79 has a corresponding taper which preserves the thickness of the wall through the region. The catheter may be constructed by coextrusion, or by one of the alternative methods of covering the tapered, reduced-thickness portion of the proximal tube, with a flexible tube, as described above. An advantage of this embodiment over the catheter shown in Figure 5 is a reduced tendency of the catheter to kink in the transition region. Also the construction provides greater column strength and torqueability in a region of the distal segment where sharp bends and turns are less likely to be encountered than at the tip region of the catheter.

### D. Chemically Hardened Surface Coating

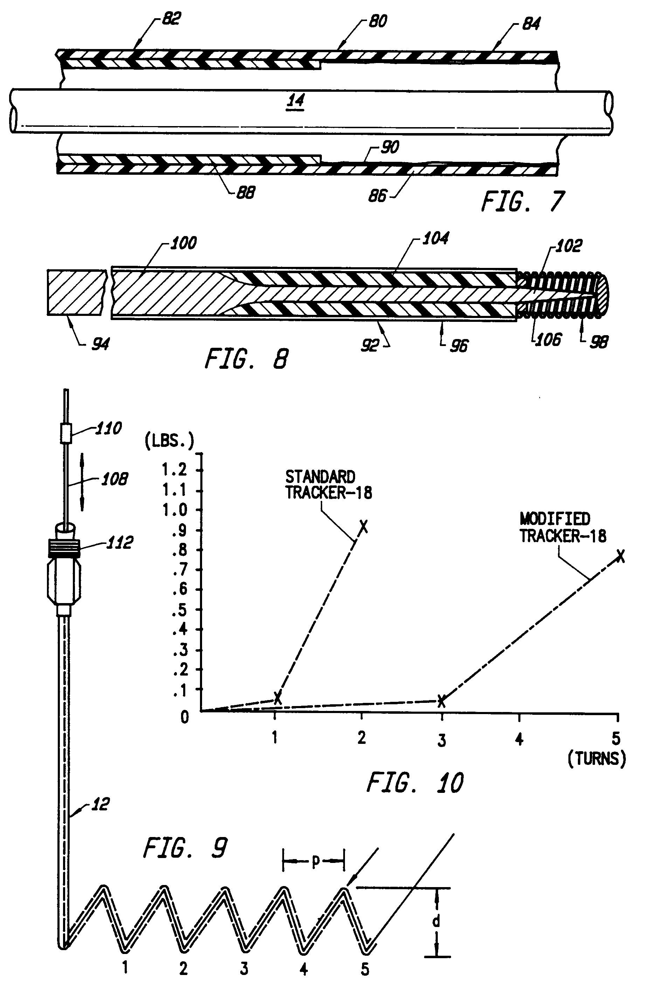

In a fourth general embodiment of the invention, shown in Figure 7, the low-friction surface coating in the distal segment of the catheter is produced by chemically hardening the inner wall surface 90 of the distal segment tube 82. One catheter embodying this feature is shown at 80 in Figure 7, which shows proximal and distal segments 82, 84, respectively, having the general construction described above. Specifically, the distal segment is formed by a distal extension of an outer tube 86 beyond the end of a stiffer, less deformable inner tube 88.

The low-friction surface 90 in the catheter is formed by chemically treating the inner surface of the distal-segment tube with a surface hardening agent, such as a polymer cross-linking agent. For example, a distal-segment tube formed of a polyurethane/acrylate copolymer may be hardened by treatment with polyisocyanate (see WO-A-86/04591 disclosing such methods of treatment). Alternatively, a hydroxylated polymer distal-segment tube, such as one made of polyvinyl alcohol, may be cross-linked using ethyl silicate (see EP-A-0 102 315). Generally, the depth of cross-linking within the distal-segment tube can be controlled by passing a solution of the cross-linking reagent through the tube as it is extruded, or heated. The degree of hardness may be controlled by techniques which are well-known in the polymer field.

It will be appreciated that the hardened surface coating can also be achieved by coating the wall surface with a hard-surface coating, such as a metal, polymer, or graphitic coating, applied by known sputtering, plating or coating techniques.

The low-friction surface or coat 90 may be constructed in a variety of different configurations. However, it is most preferable to keep the surface 90 with a highly glossed or smooth configuration in order to take maximum advantage of the anti-friction coating. By providing the hardened, highly glossed, smooth surface 90 it is possible to obtain the essential object of the invention which is to reduce any sticking or jamming of the distal end of the guidewire against the surface 90 when the guidewire and tubular catheter must be moved relative to each other and both are in a sharply bent configuration. The distal end of the guidewire should be comprised of a material which takes into consideration the composition of the surface 90. Clearly, the two materials, that is the material making up the surface 90 and the material making up the surface of the distal end of the guidewire, should be such as to avoid sticking--that is avoid any friction between the two surfaces and thus provide the lowest possible coefficient of friction when these two surfaces are forced against each other and move across each other.

### E. Guidewire Construction

Figure 8 shows one preferred type of a guidewire 92 which can be used in a catheter device of the present invention. Guidewires and their construction have been described in detail in US-A-4,832,047 which is incorporated herein by reference to disclose such. Briefly, the guidewire includes a flexible proximal section 94 having a typical length between about 40-250 cm, an intermediate section 96 having a length between about 0,04-0,15 cm (15-60 mils), and a most flexible (relative to the other sections) distal end section 98 whose length is between about 1-10 cm. The core 100 and 102 is tapered from the proximal-section diameter 100 down to a reduced diameter 102 which is preferably about 0,01-0,05 cm (4-20 mils) and between about 10%-50% of the core's proximal segment diameter.

Two segments 100 and 102 making up the core of the intermediate section of the wire are covered along their length by a flexible polymer covering 104, which functions to provide a smooth outer surface of the intermediate section, and to increase the column strength of the reduced-diameter core in the intermediate section. Covering 104 is preferably formed of a polymer, such as Teflon-like material, polyolefin, or polyurethane which can be bonded or otherwise tightly affixed to the core wire.

The distal section portion of the core is fully or partially encased in a flexible sleeve 106. The sleeve shown in Figure 8 is a soft, flexible helical coil which is formed conventionally, e.g., as described above. It is noted that the portion of the guidewire over which the flexible distal segment of the catheter is advanced is predominantly the smooth-walled proximal section, rather than the coil-encased distal wire segment.

The guidewire may be constructed to include a plurality of different types of coatings on its outer surface. For example, the guidewire may be constructed so that the outer surface has structural configurations which aid in preventing the jamming or locking of this outer surface against the inner tubular wall surface of the catheter tube. Further, the outer coating of the guidewire may be comprised of materials which aid in reducing the frictional resistance between the outer surface of the guidewire and the inner tubular surface when these two surfaces are moving relative to each other. The surfaces of the two components, that is the outer surface of the guidewire and the inner tubular wall surface of the catheter, are preferably each constructed with the structural configurations and material compositions of the other in mind so as to obtain the best possible results. More specifically, the structural features and materials used in each component are preferably chosen so as to obtain the least amount of sticking, jamming or locking of the guidewire against the inner tubular surface of the catheter.

### I. Test Characteristics

The catheter of the present invention is designed for advancement along a guidewire which has been placed, by movement along a tortuous path, in a highly convoluted bent and/or coiled configuration. Figure 9 illustrates a test configuration for measuring the ability of a catheter of the invention to be advanced along a guidewire containing a series of helical windings.

The guidewire used in this test was a 0,04 cm (14-mil) stainless steel mandrel 108 having a total length of 175 cm. The proximal end of the mandrel was clamped to the upper jaw 110 of a conventional tensile test device designed to measure the tensile force applied between two jaws 110 and 112, as the jaws are moved relatively toward or away from one another. A distal end portion of the mandrel was formed into a helix 114 having five windings which are numbered 1, 2, 3, 4 and 5 in Figure 9. The helix diameter d was about 10 mm, and the helix pitch p, about 5 mm.

Each catheter (of the type shown in Figure 2A) that was tested was flushed with saline and back loaded over the mandrel until the catheter's distal end was just upstream of the coiled portion of the mandrel. The proximal catheter end was locked in jaw 112, as shown, and this jaw was moved downwardly with respect to the stationary jaw 110, to advance the distal segment of the catheter over the coiled portion of the mandrel. The tensile force between the two jaws as this movement occurs was measured conventionally, and the force data were recorded on a chart recorder, along with the position of the catheter's tip on the guidewire coil.

The test catheter was one having a 135 cm proximal segment, a 20 cm flexible distal segment, a lumen diameter of 0,06 cm (22 mils), and a 0,06 cm (22 mils) inner diameter closely wound platinum coil sleeve extending along the entire length of the distal segment. A control catheter had the same construction, but without the distal segment coil sleeve.

Figure 10 is a graph which plots the force, in pounds, applied between the jaws in the test device, with respect to the position the distal end of the catheter as advanced along the helix. The force curve of the catheter of the present invention is indicated by dash-dot line (the line to the right), and that of the control catheter, by dashed line. The data plotted represents the average of three different test runs for each catheter device.

As seen from Figure 10, the catheter of the present invention was able to be advanced over three coil windings with very little force (less than about 0.1 lb), with linearly increasing force being required for advance between the third and fifth coil windings. The catheter could be easily advanced over the five-winding helix and only stopped when the proximal segment of the catheter reached the coiled section of the mandrel.

The force curve of the control catheter, shown in dashed lines, indicates the much greater resistance which is encountered in advancing this catheter over a coiled guidewire segment. The catheter could be advanced only over one coil winding at low force, with a sharp increase in force required in advancing along the second winding. The catheter could not be advanced over two complete guidewire windings.

Additional tests carried out in support of the invention show that the guidewire can be advanced easily over a guidewire loop having a loop diameter of 2 mm. By contrast, the smallest loop over which the control catheter could be advanced was a 4 mm diameter loop.

### II. Operation

The operation of the catheter and catheter device of the invention, in accessing a target region along a tortuous, small-vessel path will be described now with reference to Figure 11, which shows a region of soft target tissue 120 such as brain tissue, which includes a portion of a small-vessel, tortuous pathway which must be traversed in reaching a selected target site (not shown). The region shown contains vessel 122 which branches into vessel 124, and a vessel 126 which branches from the lower portion of vessel 124. The vessels may have diameters typically between about 2-5 mm or less, and the bends at both of the junctions connecting vessel 122 with the lower portion of vessel 124, and vessel 124 with vessel 126 are greater than 90 degrees.

To reach region 120, the guidewire and catheter, shown at 46, 14, respectively, are first threaded as a unit from an external access site through the vasculature to a region adjacent, but not into the tortuous path region of the target tissue. This is done in the usual case where the catheter must pass through the cardiac aorta by first placing a relatively large-diameter guiding catheter (e.g., about 0,1 cm (40 mils) inner diameter) from the access site through the aorta and toward the target site. The catheter and guidewire are then threaded through the guiding catheter past the aorta, where large-vessel diameters and high blood flow volumes make it difficult or impossible to control the movement and position of the catheter.

Once the catheter device is beyond the guiding catheter, into the target tissue, the catheter and guidewire are controlled to move toward the target site. Specifically, the guidewire is advanced independently along the tortuous path in the target tissue, according to standard wire manipulations, which include rotating or torquing the wire at each bend, to orient the wire toward the next vessel in the pathway.

For example, in Figure 11, the wire, when it reaches the junction of vessels 122, 124, is torqued to orient the wire (which has a distal bend) downwardly, and the wire is then advanced with respect to the catheter, into vessel 124. When the next vessel junction is reached, the wire is torqued in the opposite direction, and advanced from vessel 124 into 126. At some point --for example, when the wire has been advanced a total of 2-8 cm ahead of the catheter -- the catheter is then advanced over the wire, to thread the catheter up to a point near the distal end of the guidewire.

The bent region of catheter 40 and guidewire 14 in Figure 11 are shown in enlarged sectional view in Figure 12. The catheter embodiment shown is that described with respect to Figure 4B; however, the following discussion applies equally to all of the embodiments described herein). As seen in Figure 12, the spaced particles on the outer sides of each bend are engaged with the guidewire, as the catheter is advanced over the guidewire, to provide a low-friction contact between the wire and catheter lumen. As detailed above, the reduced friction, due at least in part to the non-deformability of the particles, allows the catheter to be advanced over sharper bends, and with substantially less axial force, than a flexible polymer tube alone.

Once the catheter has been advanced to the target site, the guidewire is withdrawn to allow a fluid material to be injected into the site. The injected material may include: (1) radio-opaque agents for viewing blood vessel anatomy and blood flow characteristics in the target region; (2) vaso-occlusive agents, such as a suspension of collagen fibers which can be used to produce small-artery vaso-occlusion in the tissue region supplied by the target vessel; and (3) pharmacological agents, such as anti-tumor drugs which are effective against identified disease states at the target site.

From the foregoing, it can be appreciated how various objects and features of the invention are met. The novel catheter construction described herein allows for tracking along a tortuous path over a guidewire containing multiple loops or bends whose small radii of curvature, with substantially reduced axial force needed in advancing the wire. This feature allows the catheter access to a variety of deep tissue target sites which have been inaccessible heretofore because of inability to advance the catheter along the guidewire and/or locking of catheter with the guidewire in regions of sharp bends.

In several of the embodiments, the inertial mass contributed by the low-friction surface coating is relatively small, and thus has little effect on the ability of the catheter to track the guidewire over sharp bends or turns in the tapered region of the guidewire.

Where the flexible surface structure is a radio-opaque material, such as gold, platinum, or tungsten wire, the distal segment of the catheter can be readily visualized fluoroscopically, allowing the user to view the extent of catheter advance over a guidewire and thereby better control the catheter placement operation. Alternatively, the distal-segment tube can be provided with radio-opaque banding or embedded radio-opaque material to allow fluoroscopic viewing of the distal segment during use.

The catheter can be easily manufactured using conventional catheter production methods, including coil winding and polymer tube extrusion methods.

While the catheter and catheter device have each been described with reference to specific embodiments thereof, it should be understood by those skilled in the art that various changes may be made and equivalence may be substituted without departing from the scope of the invention. In addition, many modifications may be made to adapt a particular situation, material, composition of matter, process, process step or steps, to the scope of the present invention. All such modifications are intended to be within the scope of the claims appended hereto.

## Claims

1. A catheter for use in combination with a guidewire (14) for accessing a target site in an internal body tissue, from an external body site to the internal body tissue, and along a tortuous, small-vessel pathway within the tissue, said catheter comprising:
an elongate tubular member (12) having proximal (18) and distal ends, and an inner lumen (22) extending between these ends, the lumen having a diameter which is no greater than about 0,1cm (40 mils), said member including a relatively rigid proximal segment (24) and a relatively flexible distal segment (26, 66, 84) at least about 5cm long which is adapted for tracking the guidewire (14) along such tortuous path, said distal segment (26) including a flexible, relatively deformable, polymer distal-segment tube;
characterised in that the distal segment (26) further includes a relatively non-deformable internal surface means (38, 38A, 42) carried on the inner surface (56) of the distal-segment tube, for providing substantially uninterrupted reduced friction contact with the guidewire (14) as the distal segment of the catheter is advanced over a looped or bent region of the guidewire.

2. The catheter of claim 1, wherein said distal segment (26) is composed of low-density polyethylene.

3. The catheter of claim 1 or claim 2, wherein said surface means includes a sleeve (38, 38A, 42).

4. The catheter of claim 3, wherein the sleeve (38, 42) is a braided-filament sleeve.

5. The catheter as claimed in claim 4, wherein the braided sleeve (38, 42) is comprised of an anti-friction material.

6. The catheter of claim 4 or claim 5, wherein the filaments forming said braided sleeve (38, 42) are composed of material selected from the group of elements consisting of: platinum, acrylic, nylon and Kevlar.

7. The catheter of any one of claims 4 to 6, wherein the pitch of braid (42) is reduced in a direction progressing toward the distal end (44) of the catheter, to achieve a greater flexibility in the distal catheter end.

8. The catheter as claimed in claim 3, wherein the sleeve is a wire coil (38A).

9. The catheter as claimed in claim 8, wherein the wire coil (38A) is comprised of an anti-friction material.

10. The catheter of any one of claims 3 to 9, wherein said distal-segment tube further includes a distal extension (40) composed of a flexible, relatively deformable, polymer tube which lacks said sleeve (38, 38A, 42).

11. The catheter of claim 1 or claim 2, wherein said surface means includes an array of substantially non-deformable smooth-surfaced particles (48) carried on the inner surface of the distal-segment tube.

12. The catheter of claim 11, wherein the particles (48) are carbon particles attached to the inner surface (56) of the distal-segment tube by a binder (50).

13. The catheter of claim 1 or claim 2, wherein said surface means includes a thin-walled extension of said proximal segment (62, 74) forming an inner coaxial lining (70) of the distal segment (66, 78).

14. The catheter of claim 13, wherein the proximal segment (62) is formed of a polymer selected from the group consisting of high-density polyethylene, Teflon, polypropylene, and polyurethane, and the distal segment surrounding the inner coaxial lining is low-density polyethylene.

15. The catheter of claim 1 or claim 2, wherein said surface means is a chemically hardened polymer surface coat formed by cross-linking the inner wall surface (90) of the distal segment (84).

16. The catheter of claim 1 or claim 2, wherein said surface means is a wire coil (38A) having an inner diameter of between about 0,02-0,08cm (8-30 mils).

17. The catheter of claim 16, wherein the coil (38A) is formed of windings having a given diameter, and the spacing between adjacent coil windings is between about 30-70 percent of the given winding diameter.

18. The catheter of claim 16 or claim 17, wherein the coil (38A) is formed from platinum, tungsten, gold, or alloys thereof.

19. The catheter of claim 1 or claim 2, wherein said surface means includes a series of axially spaced rings embedded in the inner surface of said distal-segment tube, said rings having relatively non-deformable surfaces.

20. The catheter of claim 1 or claim 2, wherein said surface means includes an array of relatively non-deformable particles (48) partially embedded within the inner surface of the distal-segment tube.

21. The catheter of any one of claims 1-12 and 15-30, wherein said proximal segment is formed of inner and outer coaxial tubes, one of which is relatively stiff, and one of which is relatively flexible, and said distal-segment tube is a distal extension of the relatively flexible tube.

22. The catheter of claim 21, when dependent on either of claims 1 and 2 only, wherein the relatively flexible tube is the outer of the two coaxial tubes, said surface means is a flexible wire coil, said coil has a proximal end which is received in a distal end of the inner tube, and the remainder of the coil is encased in the outer, distal end portion of the more flexible tube which forms the distal-segment tube.

23. The catheter of any one of the preceding claims, wherein said proximal segment (24) is relatively longer than said distal segment (26, 66, 84).

24. The catheter of any one of the preceding claims, wherein the distal segment (26, 66, 84) is able to be advanced over a guidewire loop having a 2mm diameter.

25. The catheter of any of claims 8, 9, 16-18 and 22, wherein the wire coil has an inner diameter of about 0,02-0,08cm (8-30 mils).

26. The catheter of claim 1, 2 or 11, wherein said surface means (48) are embedded in the inner surface (56) of the distal segment tube.

27. The catheter of any one of the preceding claims, in combination with a guidewire (14), the guidewire having a distal end region (98) which is encased in a wire coil (106), and a smooth-walled intermediate region (96) adjacent the distal end region (98) which is arranged to contact the distal segment of the catheter during a catheter placement operation.

28. The catheter of claim 27, wherein the smooth-walled intermediate region (96) has an outer surface (104) formed by a lubricious polymer material.

29. The catheter of claim 27 or claim 28, wherein the inner diameter of said distal segment is between about 0,005-0,02cm (2-7 mils) larger than the diameter of the wire's smooth-walled intermediate region (96).

30. The catheter of any one of claims 1 to 26 in combination with a guidewire (14), the guidewire having proximal and distal ends and a wire diameter no greater than about 0,03cm (10 mils).

## Patentansprüche

1. Katheter zur Verwendung in Kombination mit einem Führungsdraht (14), um von einer äußeren Stelle am Körper zu einem inneren Körpergewebe und längs eines gewundenen Weges in einem kleinen Gefäß in dem Gewebe zu einer Zielstelle in einem inneren Körpergewebe zu gelangen, wobei der Katheter folgendes umfaßt:
ein langgestrecktes rohrförmiges Element (12) mit einem proximalen (18) und einem distalen Ende und einem inneren Lumen (22), das sich zwischen diesen Enden erstreckt, wobei das Lumen einen Durchmesser aufweist, der nicht größer ist als etwa 0,1 cm (40 Millizoll), wobei das Element ein relativ steifes proximales Segment (24) und ein relativ flexibles distales Segment (26, 66, 84) mit einer Länge von mindestens etwa 5 cm aufweist, das den Führungsdraht (14) längs eines solchen gewundenen Weges führen kann, wobei das distale Segment (26) ein flexibles, relativ verformbares, polymeres Röhrchen am distalen Segment aufweist;
dadurch gekennzeichnet, daß das distale Segment (26) des weiteren eine relativ unverformbare Innenseiteneinrichtung (38, 38A, 42) umfaßt, die auf der Innenseite (56) des Röhrchens am distalen Segment angeordnet ist und einen im wesentlichen ununterbrochenen, reibungsgeminderten Kontakt mit dem Führungsdraht (14) herstellt, wenn das distale Segment des Katheters über einen gewundenen oder gebogenen Bereich des Führungsdrahtes geführt wird.

2. Katheter nach Anspruch 1, bei dem das distale Segment (26) aus Polyethylen niedriger Dichte besteht.

3. Katheter nach Anspruch 1 oder Anspruch 2, bei dem die Innenseiteneinrichtung eine Hülse (38, 38A, 42) umfaßt.

4. Katheter nach Anspruch 3, bei dem die Hülse (38, 42) eine Hülse aus geflochtenen Filamenten ist.

5. Katheter nach Anspruch 4, bei dem die geflochtene Hülse (38, 42) aus einem reibungsmindernden Material besteht.

6. Katheter nach Anspruch 4 oder Anspruch 5, bei dem die die geflochtene Hülse (38, 42) bildenden Filamente aus einem Material bestehen, das ausgewählt ist aus der Gruppe von Elementen umfassend Platin, Acryl, Nylon und Kevlar.

7. Katheter nach einem der Ansprüche 4 bis 6, bei dem die Flechtdichte (42) in einer Richtung zum distalen Ende (44) des Katheters hin vermindert ist, um eine größere Flexibilität am distalen Ende des Katheters zu erreichen.

8. Katheter nach Anspruch 3, bei dem die Hülse eine Drahtspule (38A) ist.

9. Katheter nach Anspruch 8, bei dem die Drahtspule (38A) aus einem reibungsmindernden Material besteht.

10. Katheter nach einem der Ansprüche 3 bis 9, bei dem das Röhrchen am distalen Segment des weiteren eine distale Verlängerung (40) umfaßt, die aus einem flexiblen, relativ verformbaren Röhrchen aus Polymer besteht, an dem die Hülse (38, 38A, 42) fehlt.

11. Katheter nach Anspruch 1 oder Anspruch 2, bei dem die Innenseiteneinrichtung eine Anordnung von im wesentlichen unverformbaren Teilchen (48) mit glatter Oberfläche umfaßt, die auf der Innenseite des Röhrchens am distalen Segment angeordnet sind.

12. Katheter nach Anspruch 11, bei dem die Teilchen (48) Kohlenstoffteilchen sind, die mit einem Binder (50) auf der Innenseite (56) des Röhrchens am distalen Segment befestigt sind.

13. Katheter nach Anspruch 1 oder Anspruch 2, bei dem die Innenseiteneinrichtung eine dünnwandige Verlängerung des proximalen Segments (62, 74) umfaßt, die eine innere koaxiale Deckschicht (70) an dem distalen Segment (66, 78) bildet.

14. Katheter nach Anspruch 13, bei dem das proximale Segment (62) aus einem Polymer besteht, das ausgewählt ist aus der Gruppe umfassend Polyethylen hoher Dichte, Teflon, Polypropylen und Polyurethan, und bei dem das die innere axiale Deckschicht umschließende distale Segment aus Polyethylen niedriger Dichte besteht.

15. Katheter nach Anspruch 1 oder Anspruch 2, bei dem die Innenseiteneinrichtung ein Überzug aus Polymer mit einer chemisch gehärteten Oberfläche ist, der durch Quervernetzung auf der inneren Wandseite (90) des distalen Segments (84) ausgebildet wird.

16. Katheter nach Anspruch 1 oder 2, bei dem die Innenseiteneinrichtung eine Drahtspule (38A) mit einem Innendurchmesser von etwa 0,02 - 0,08 cm (8 - 30 Millizoll) ist.

17. Katheter nach Anspruch 16, bei dem die Spule (38A) aus Wicklungen mit einem vorgegebenen Durchmesser besteht und der Abstand zwischen benachbarten Spulenwicklungen etwa 30 - 70 Prozent des vorgegebenen Wicklungsdurchmessers beträgt.

18. Katheter nach Anspruch 16 oder Anspruch 17, bei dem die Spule (38A) aus Platin, Wolfram, Gold oder Legierungen aus denselben besteht.

19. Katheter nach Anspruch 1 oder Anspruch 2, bei dem die Innenseiteneinrichtung eine Reihe von axial voneinander beabstandeten Ringen umfaßt, die in die Innenseite des Röhrchens am distalen Segment eingebettet sind, wobei die Ringe eine relativ unverformbare Oberfläche aufweisen.

20. Katheter nach Anspruch 1 oder 2, bei dem die Innenseiteneinrichtung eine Anordnung von relativ unverformbaren Teilchen (48) aufweist, die zum Teil in die Innenseite des Röhrchens am distalen Segment eingebettet sind.

21. Katheter nach einem der Ansprüche 1 - 12 und 15 - 30, bei dem das proximale Segment aus inneren und äußeren koaxialen Röhrchen besteht, von denen eines relativ steif ist und eines relativ flexibel ist, und bei dem das Röhrchen am distalen Ende eine distale Verlängerung des relativ flexiblen Röhrchens ist.

22. Katheter nach Anspruch 21, wenn nur auf einen der Ansprüche 1 und 2 bezogen, bei dem das relativ flexible Röhrchen das äußere der beiden koaxialen Röhrchen ist, die Innenseiteneinrichtung eine flexible Drahtspule ist, die Spule ein proximales Ende aufweist, das in einem distalen Ende des inneren Röhrchens aufgenommen ist, und der Rest der Spule in dem äußeren distalen Endabschnitt des flexibleren Röhrchens eingeschlossen ist, welches das Röhrchen am distalen Segment bildet.

23. Katheter nach einem der vorhergehenden Ansprüche, bei dem das proximale Segment (24) relativ länger ist als das distale Segment (26, 66, 84).

24. Katheter nach einem der vorhergehenden Ansprüche, bei dem das distale Segment (26, 66, 84) über eine Führungsdrahtschlinge mit einem Durchmesser von 2 mm geführt werden kann.

25. Katheter nach einem der Ansprüche 8,9, 16 - 18 und 22, bei dem die Drahtspule einen Innendurchmesser von etwa 0,02 - 0,08 cm (8 - 30 Millizoll) besitzt.

26. Katheter nach Anspruch 1, 2 oder 11, bei dem die Innenseiteneinrichtungen (48) in die Innenseite (56) des Röhrchens am distalen Segment eingebettet sind.

27. Katheter nach einem der vorhergehenden Ansprüche in Kombination mit einem Führungsdraht (14), wobei der Führungsdraht einen distalen Endbereich (98) aufweist, der in einer Drahtspule (106) eingeschlossen ist, sowie einen glattwandigen Zwischenbereich (96) nahe an dem distalen Endbereich (98), der so angeordnet ist, daß er bei einer Katheterpositionierung mit dem distalen Segment des Katheters in Berührung kommt.

28. Katheter nach Anspruch 27, bei dem der glattwandige Zwischenbereich (96) eine Außenseite (104) aufweist, die aus einem gleitfähigen polymeren Material ausgebildet ist.

29. Katheter nach Anspruch 27 oder Anspruch 28, bei dem der Innendurchmesser des distalen Segments um etwa 0,005 - 0,02 cm (2 - 7 Millizoll) größer ist als der Durchmesser des glattwandigen Zwischenbereiches (96) des Drahtes.

30. Katheter nach einem der Ansprüche 1 bis 26 in Kombination mit einem Führungsdraht (14), wobei der Führungsdraht ein proximales und ein distales Ende sowie einen Drahtdurchmesser von höchstens etwa 0,03 cm (10 Millizoll) aufweist.

## Revendications

1. Cathéter destiné à être utilisé en combinaison avec un fil-guide (14) pour accéder à un site-cible dans un tissu interne du corps, depuis un lieu extérieur du corps jusqu'au tissu interne du corps, et en suivant le trajet tortueux de petits vaisseaux dans le tissu, ledit cathéter comprenant:
un élément tubulaire allongé (12) ayant des extrémités proximale (18) et distale, et un conduit intérieur (22) s'étendant entre ces extrémités, le conduit ayant un diamètre maximal d'environ 0,1 cm (40 mils), ledit élément comportant un segment proximal relativement rigide (24) et un segment distal relativement souple (26, 66, 84) d'au moins environ 5 cm de longueur servant à faire passer le fil-guide (14) le long dudit trajet tortueux, ledit segment distal (26) comportant un tuyau souple en polymère, relativement déformable, de segment distal;
caractérisé en ce que le segment distal (26) comporte en outre un moyen de surface interne relativement non déformable (38, 38A, 42) porté par la surface intérieure (56) du tuyau de segment distal, pour réaliser un contact à frottement réduit sensiblement ininterrompu avec le fil-guide (14) à mesure qu'on fait avancer le segment distal du cathéter sur une partie courbe ou coudée du fil-guide.

2. Cathéter selon la revendication 1, dans lequel ledit segment distal (26) est en polyéthylène basse densité.

3. Cathéter selon la revendication 1 ou la revendication 2, dans lequel ledit moyen de surface comporte un manchon (38, 38A, 42).

4. Cathéter selon la revendication 3, dans lequel le manchon (38, 42) est un manchon filamentaire tressé.

5. Cathéter selon la revendication 4, dans lequel le manchon tressé (38, 42) est en matière antifriction.

6. Cathéter selon la revendication 4 ou la revendication 5, dans lequel les filaments formant ledit manchon tressé (38, 42) sont en matière choisie dans le groupe d'éléments comprenant: le platine, l'acrylique, le Nylon et le Kevlar.

7. Cathéter selon l'une quelconque des revendications 4 à 6, dans lequel le pas de la tresse (42) diminue dans la direction de l'extrémité distale (44) du cathéter, pour réaliser une plus grande souplesse à l'extrémité distale du cathéter.

8. Cathéter selon la revendication 3, dans lequel le manchon est un enroulement hélicoïdal de fil (38A).

9. Cathéter selon la revendication 8, dans lequel l'enroulement hélicoïdal de fil (38A) est en matière antifriction.

10. Cathéter selon l'une quelconque des revendications 3 à 9, dans lequel ledit tuyau de segment distal comporte en outre un prolongement distal (40) constitué par un tuyau souple, relativement déformable, en polymère qui dépasse dudit manchon (38, 38A, 42).

11. Cathéter selon la revendication 1 ou la revendication 2, dans lequel ledit moyen de surface comporte un ensemble de particules (48) à surface lisse sensiblement indéformables portées par la surface intérieure du tuyau de segment distal.

12. Cathéter selon la revendication 11, dans lequel les particules (48) sont des particules de carbone fixées par un liant (50) à la surface intérieure (56) du tuyau de segment distal.

13. Cathéter selon la revendication 1 ou la revendication 2, dans lequel ledit moyen de surface comporte un prolongement à paroi mince dudit segment proximal (62, 74) formant un chemisage coaxial intérieur (70) du segment distal (66, 78).

14. Cathéter selon la revendication 13, dans lequel le segment proximal (72) est en polymère choisi dans le groupe comprenant le polyéthylène haute densité, le Teflon, le polypropylène et le polyuréthane, et le segment distal entourant le chemisage coaxial intérieur est en polyéthylène basse densité.

15. Cathéter selon la revendication 1 ou la revendication 2, dans lequel ledit moyen de surface est un revêtement de surface en polymère durci par voie chimique, formé par réticulation de la surface de paroi intérieure (90) du segment distal (84).

16. Cathéter selon la revendication 1 ou la revendication 2, dans lequel ledit moyen de surface est un enroulement hélicoïdal (38A) de fil ayant un diamètre intérieur compris environ entre 0,02 et 0,08 cm (8 et 30 mils).

17. Cathéter selon la revendication 16, dans lequel l'enroulement (38A) est constitué par des spires d'un diamètre donné, et l'espacement entre les spires contiguës de l'enroulement est compris environ entre 30 et 70 pour cent du diamètre donné des spires.

18. Cathéter selon la revendication 16 ou la revendication 17, dans lequel l'enroulement (38A) est en platine, en tungstène, en or ou en alliages de ceux-ci.

19. Cathéter selon la revendication 1 ou la revendication 2, dans lequel ledit moyen de surface comporte une série d'anneaux espacés axialement, noyés dans la surface intérieure dudit tuyau de segment distal, lesdits anneaux ayant des surfaces relativement indéformables.

20. Cathéter selon la revendication 1 ou la revendication 2, dans lequel ledit moyen de surface comporte un ensemble de particules relativement indéformables (48) partiellement noyées dans la surface intérieure du tuyau de segment distal.

21. Cathéter selon l'une quelconque des revendications 1 à 12 et 15 à 20, dans lequel ledit segment proximal est constitué par des tuyaux intérieur et extérieur coaxiaux, dont l'un est relativement rigide, et dont l'un est relativement souple, et ledit tuyau de segment distal est un prolongement distal du tuyau relativement souple.

22. Cathéter selon la revendication 21, dépendant uniquement de l'une ou l'autre des revendications 1 et 2, dans lequel le tuyau souple est celui des deux tuyaux coaxiaux qui est extérieur, ledit moyen de surface est un enroulement hélicoïdal souple de fil, ledit enroulement hélicoïdal a une extrémité proximale reçue dans une extrémité distale du tuyau intérieur, et le reste de l'enroulement hélicoïdal est logé dans la partie formant extrémité extérieure distale du tuyau plus souple qui forme le tuyau de segment distal.

23. Cathéter selon l'une quelconque des revendications précédentes, dans lequel ledit segment proximal (24) est relativement plus long que ledit segment distal (26, 66, 84).

24. Cathéter selon l'une quelconque des revendications précédentes, dans lequel on peut faire avancer le segment distal (26, 66, 84) sur une boucle de fil-guide de 2 mm de diamètre.

25. Cathéter selon l'une quelconque des revendications 8, 9, 16 à 18 et 22, dans lequel l'enroulement hélicoïdal de fil a un diamètre intérieur d'environ 0,02 à 0,08 cm (8 à 30 mils).

26. Cathéter selon la revendication 1, 2 ou 11, dans lequel lesdits moyens de surface (48) sont noyés dans la surface intérieure (56) du tuyau de segment distal.

27. Cathéter selon l'une quelconque des revendications précédentes, associé à un fil-guide (14), le fil-guide ayant une partie d'extrémité distale (98) logée dans un enroulement hélicoïdal (106) de fil, et une partie intermédiaire (96) à paroi lisse contigue à la partie d'extrémité distale (98) et agencée pour venir au contact du segment distal du cathéter pendant une opération de mise en place de cathéter.

28. Cathéter selon la revendication 27, dans lequel la partie intermédiaire (96) à paroi lisse a une surface extérieure (104) en polymère à action de lubrification.

29. Cathéter selon la revendication 27 ou la revendication 28, dans lequel le diamètre intérieur dudit segment distal est plus grand d'environ 0,005 à 0,02 cm (2 à 7 mils) que le diamètre de la partie intermédiaire (96) à paroi lisse du fil.

30. Cathéter selon l'une quelconque des revendications 1 à 26 en combinaison avec un fil-guide (14), le fil-guide ayant des extrémités proximale et distale et un diamètre de fil non supérieur à environ 0,03 cm (10 mils).
